# EUROPEAN PATENT SPECIFICATION

(11) **EP 3 955 825 B1**
(45) Date of publication and mention of the grant of the patent: **18.12.2024**
(21) Application number: 20725917.7
(22) Date of filing: 16.04.2020
(51) Int. Cl.: A61B 8/08, A61B 8/12, A61B 8/00

(54) **ENDOCAVITY PROBE AND METHOD FOR PROCESSING DIAGNOSTIC IMAGES**
ENDOKAVITÄTSSONDE UND VERFAHREN ZUR VERARBEITUNG VON DIAGNOSTISCHEN BILDERN
SONDE ENDOCAVITAIRE ET PROCÉDÉ DE TRAITEMENT D'IMAGES DIAGNOSTIQUES

(30) Priority: 17.04.2019 IT 201900005986; 17.04.2019 IT 201900005988
(43) Date of publication of application: 23.02.2022
(73) Proprietor: ELESTA S.P.A., 50041 Calenzano (FI) (IT)
(72) Inventor: MASOTTI, Leonardo, 50019 Sesto Fiorentino (FI) (IT); BRESCHI, Luca, 59021 Vaiano (PO) (IT)
(74) Representative: Mannucci, Michele
(86) International application number: PCT/IB2020/053593
(87) International publication number: WO 2020/212893

(56) References cited:
- EP-A1- 2 455 133
- US-A1- 2011 295 123
- US-A1- 2013 310 679

## Description

### TECHNICAL FIELD

The present disclosure refers to medical equipment and the related methods of use. More in particular, the present disclosure discloses innovations in ultrasound imaging equipment and related methods for image processing, especially, although not exclusively, in the field of endorectal probes for prostate surgery, and, more in general, in the field of endocavity probes for diagnosis and/or interventions on organs adjacent to ducts or cavities of a patient's body.

### BACKGROUND ART

For many prostate interventions it is useful to have available ultrasound images captured by endorectal probes.

These images are useful, for example, in biopsies and in minimally invasive prostate surgery carried out through the different available technologies, for example local hyperthermia for reducing benign prostatic hyperplasia or for ablation of malignant focal tumors, through hyperthermia techniques, use of radioactive seeds (brachytherapy), or other minimally invasive transurethral or transcutaneous techniques in transperineal area with needles dispensing light energy through fibers or other carriers, for example water vapor.

The advantages of using ultrasound images captured by means of endorectal probes are the closeness of the probe to the prostate, allowing the use of high-frequency probes for acquiring high spatial resolution tomographic images, and the possibility of having images of both transversal and longitudinal sections.

Currently, for studying the whole prostate volume, sonographers use socalled biplane probes, i.e. probes having two linear arrays of active elements: a rectilinear array parallel to the probe axis, and a curvilinear array lying on a circumference on a plane orthogonal to the probe axis. With the biplane probes the sonographers can change both the longitudinal view plane, by rotating the probe around the longitudinal axis and, analogously, the transverse view plane, by moving the probe backwards or forwards in the longitudinal axis direction.

In minimally invasive interventions for ablation of malignant focal tumors, magnetic resonance imaging is used for localizing the lesion to be treated by means of the ablation ultrasound techniques mentioned above.

Magnetic resonance images are acquired during a specific session before the operation. Guided by images obtained by merging, through a software, the previously captured MR images and the ultrasound images of the patient obtained in real-time, the doctor inserts, and manually maneuvers, through the skin or natural openings, the device supplying the treatment energy, until bringing it inside the focal lesion to be ablated. This maneuver is based on the use of merged images for "navigating" inside the human body.

US 2011/0137148 discloses a method and a device for capturing magnetic resonance images and ultrasound images and for coming the to images with one another. The device provides for an endorectal bi-functional probe with coils for generating a magnetic field, and piezo-electric elements for emitting and receiving ultrasound waves. The two images, i.e. the resonance image and the ultrasound image, are captured in the course of the same medical session. The bi-functional probe is very complex. Magnetic resonance images and ultrasound images shall be captured in different time instant, to reduce the effect of the ultrasound system on the magnetic resonance system. This makes the device very complex from the programming and controlling viewpoint. Moreover, the presence of the endorectal probe in the investigated volume represents a disturbance factor for the operation of the magnetic resonance imaging system.

It should be noted that it is necessary to merge three-dimensional images to obtain a three-dimensional image on which to choose the most appropriate sections in which to obtain the information necessary to guide the device until bringing it adequately in position in the lesion to be treated.

To capture data for constructing ultrasound three-dimensional images through the endorectal probe, it is necessary to collect the images of the whole volume of interest, capturing, and storing, them in the various scan planes. For obtaining the longitudinal images it is necessary to vary the rotation angle of the probe around the longitudinal axis thereof, so as to investigate the whole arc containing the prostate gland. The movement is done by small angular steps. In practice, this movement allows obtaining a plurality of images relating to planes belonging to a bundle of planes, all containing the longitudinal axis of the endorectal probe.

Vice versa, for obtaining the transverse images it is necessary to perform subsequent constant-pitch translations, sufficiently small to capture and to store the various images for the whole longitudinal extension containing the prostate gland. In practice, the images relate to a bundle of planes parallel to one another and orthogonal to the probe longitudinal axis.

These two groups of images are usually obtained by means of two different arrays of ultrasound sensors arranged on the same probe: the first array of sensors is parallel to the probe axis, the second array is curved and the sensors thereof are arranged along a curve line, corresponding to the intersection between the probe cylindrical surface and a plane orthogonal to the probe axis.

The images according to the two bundles of planes mentioned above are captured manually, wherein the operator rotates, or translates, the probe freehand in stepped fashion. This results in inaccuracies in the captured images, due to the irregularity of movements that, in turn, affects the quality of the ultrasound data of the investigated volume and, thus, the accuracy of the merged images (ultrasound and magnetic resonance images). This adversely affects the subsequent use of the captured images during navigation, making the treatment more complex and less safe.

Furthermore, the interventions last more time, due to the slowness of capturing the necessary images, also taking into account the need for repeating the acquisition of an image in case of wrong focusing due to the inaccuracies mentioned above. This adversely affects the intervention cost and has a greater impact on the patient, to whom larger amounts of anesthetic shall be administered, when necessary.

There is therefore a need for providing new equipment and methods, completely or partially overcoming the drawbacks of the prior art.

US2013/0310679 discloses a transurethral system for multi-modal fusion, US2011/0295123 discloses a curved linear array transducer for ultrasound imaging..

### SUMMARY OF THE INVENTION

The invention is defined by a set of ultrasound and dummy probes according to claim 1, and by a computer program which implements a method which merges images acquired with the probes according to claim 7.

### SUMMARY OF THE DISCLOSURE

A subject of the present disclosure is a set comprising: an endocavity ultrasound probe having an elongated body with a convex outer surface extending along a longitudinal extension, with at least an array of ultrasound sensors that are arranged on the outer convex curved surface facing on the outer curved convex surface and are adapted to emit and receive ultrasound waves; and a dummy probe, i.e. an element adapted to be inserted into an endocavity seat, i.e. inside a cavity of the human or animal body, and having an outer surface equal, in shape and dimension, to the ultrasound probe.

As it will be better explained below, this set allows greater accuracy in merging images of the same organ captured though different techniques, for example ultrasound images captured with the above-mentioned probe and magnetic resonance images, computerized axial tomography images or other high-resolution images. These latter may be captured by inserting the dummy probe into the patient's rectum or in other cavity where the ultrasound probe shall be inserted. The dummy probe causes a displacement and/or compression of the surrounding tissues substantially equal to that caused by the real ultrasound probe, so that the compression or deformation conditions of the organs investigated with the two imaging techniques are, in different times, substantially equal or, anyway, similar. In this way the images can be better superimposed on one another during the merging step.

Here below a system is also disclosed for merging images captured through different imaging techniques, one of which comprises the acquisition of ultrasound images, wherein the images are images of an organ of a human or animal body in vivo, with which a cavity is associated, where an endocavity ultrasound probe is inserted. The cavity may be adjacent, or anyway close, to the organ, of which images shall be captured, or it can extend through the organ, i.e. the organ can at least partially surround the cavity. In some embodiments, the method comprises a first step of providing a stored first image of the organ, deformed by means of an element having a shape corresponding to an endocavity ultrasound probe, inserted into the cavity associated with the organ, the first image being captured through a first, nonultrasound, imaging technique. In this first step, a single two-dimensional image may be captured, or preferably a series of images for constructing a three-dimensional image. The first image may be captured through a high-definition imaging system, some examples of which will be mentioned below. The method also comprises a second step of providing a stored second image of the organ, captured through the endocavity ultrasound probe inserted into the cavity associated with the organ.

The first step can be performed, for example, in a session different than that of the second step. The first image, or series of two-dimensional images for constructing a three-dimensional image, may be captured through an imaging device, for example a magnetic resonance equipment, and stored in a memory support to be used during a second session. In the second session, the second image, or series of two-dimensional images for constructing a three-dimensional image, is captured, for example, through the endocavity probe associated with an ultrasound machine. The first image, or series of images, is provided to the ultrasound machine.

The method also comprises a step of superimposing the first image on, and combining it with, the second ultrasound image.

As the first image, or series of images, has been captured after having inserted, into the cavity associated with the organ to be investigated, a body or element having the same shape and dimension as the endocavity probe, merging the two images or series of images is more accurate.

The above-mentioned element, indicated below as "dummy probe" and inserted into the cavity before capturing the first image, has a shape corresponding to an endocavity ultrasound probe, i.e. the element portion interacting with the human or animal body, of which images shall be captured, has such shape and dimension to cause, in the investigated organ(s), the same deformation caused by the endocavity probe. This means that the element or dummy probe may have, with respect to the endocavity probe, different additional parts or portions, provided that these do not alter the deformation effect on the investigated organ caused by the dummy probe with respect to the deformation effect caused by the endocavity ultrasound probe.

### BRIEF DESCRIPTION OF THE DRAWING

The invention will be better understood by following the description below and the attached drawing, showing a non-limiting embodiment of the invention. More specifically, in the drawing:
Figs. 1A, 1B, 1C are axonometric views of a probe according to the invention in different modes of excitation of the ultrasound sensors;
Fig. 2 is a schematic view of a use of the probe;
Fig. 3 is a schematic cross-section showing the use of the probe;
Fig. 4 is an axonometric view of a dummy probe;
Fig. 5 is a flow diagram summarizing the operating steps;
Figs. 6A-6D are schematized images captured through magnetic resonance and ultrasound probe, as well as the merging thereof;
Fig. 7 is a side view of a linear array probe and the support thereof in an embodiment;
Fig. 8 shows a view according to VIII-VIII of Fig. 7;
Figs. 9A and 9B are axonometric views of the probe and the support thereof in two distinct angular positions;
Fig. 10 is an axonometric view of a biplane probe and the sensors thereof; and
Fig. 11 shows a diagram illustrating a method for controlling the temporal delays of excitation of an array of ultrasound sensors.

### DETAILED DESCRIPTION

In the following description, specific reference will be made to endorectal probes to be used in minimally invasive prostate surgery and to methods using the images captured through these probes. However, the novel features disclosed herein with reference to an endorectal probe can be also used in other applications, for producing endocavity probes in general, i.e. probes adapted to be used in cavities of the human or animal body, for constructing three-dimensional images of an organ, a tissue, or a generic portion of the human or animal body, by capturing data on ultrasound signal acquisition planes, in particular a plurality of planes of a bundle of planes containing the probe axis and/or a plurality of parallel planes orthogonal to the probe axis.

Therefore, what disclosed herein can be conceptually applied also to navigation for performing tumor ablation treatments in all cases when the lesions to be treated are close to the inner wall of a tubular structure of organs creating a natural communication channel with the outside of the patient's body, wherein into said tubular structure an ultrasound probe or other endoscopic structure can be introduced, and wherein the lesions can be detected and localized through a high-definition imaging technique (imaging diagnostics) for example, although not exclusively, through magnetic resonance.

Two different types of probes will be described below, as well as their applications to the disclosed method: a first novel electronic scanning probe, provided with a two-dimensional matrix of electronically controlled piezo-electric elements, i.e. of ultrasound sensors; and a manually controlled second biplane probe representing the prior art, with two linear arrays of piezo-electric elements, i.e. ultrasound sensors.

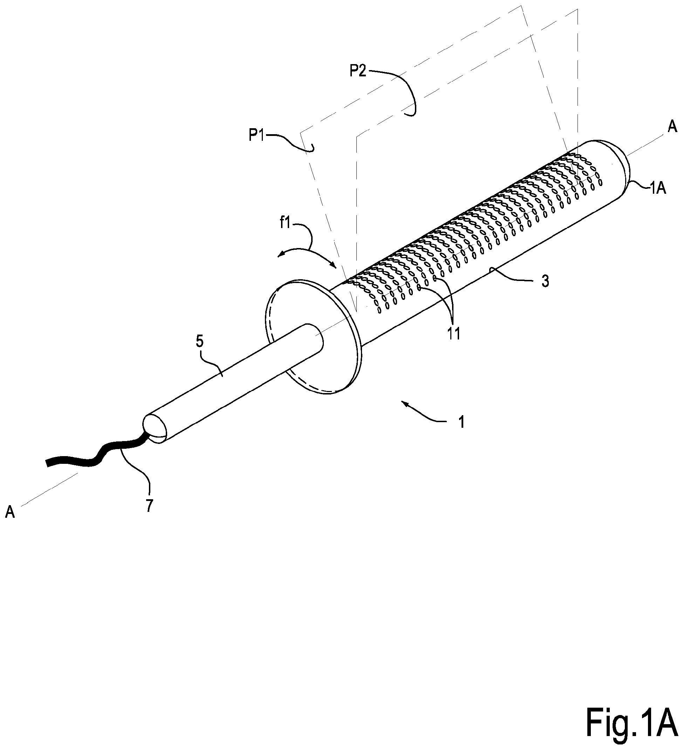

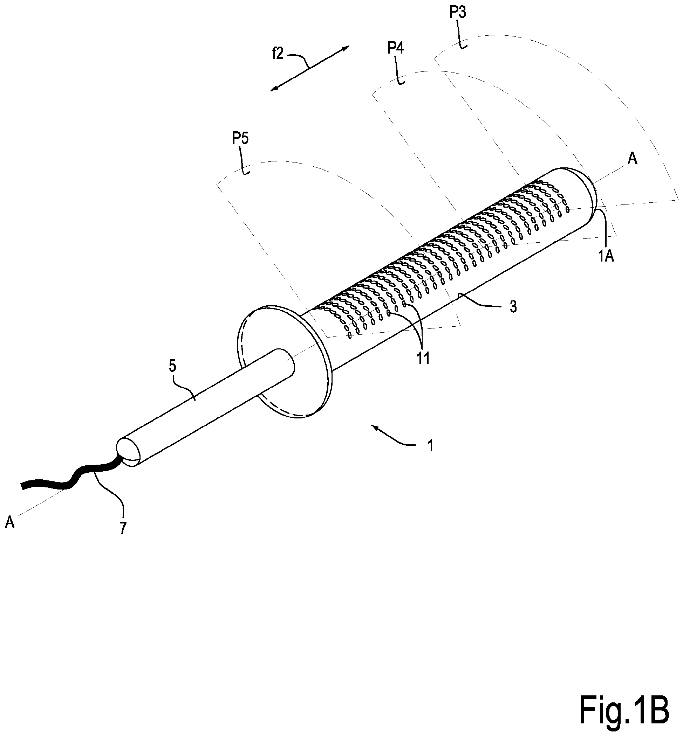

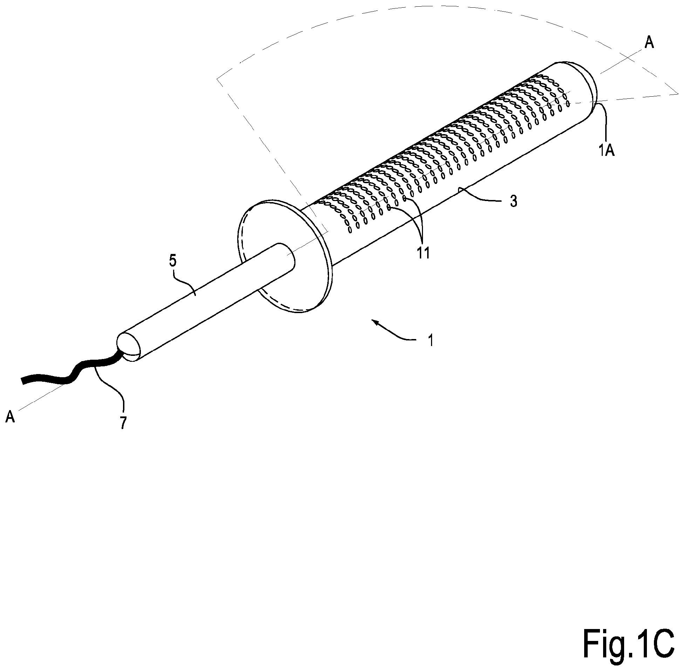

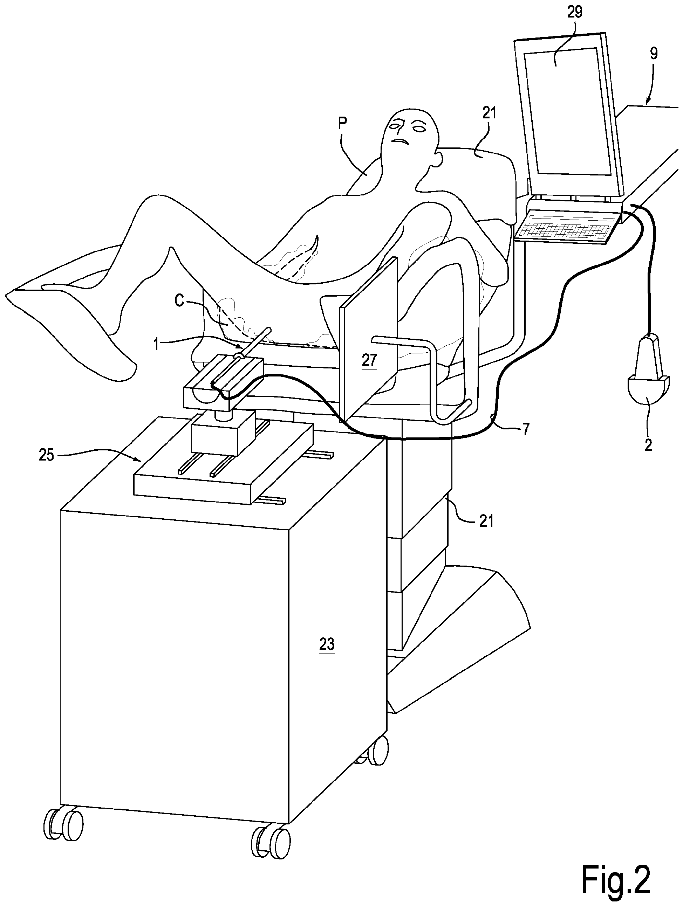

Now reference will be made to the attached drawing, and especially to Figs. 1 to 4; Fig. 1 shows an embodiment of an endorectal ultrasound probe 1. The ultrasound probe 1 has an elongated body 3 with a longitudinal axis A-A. Advantageously, the elongated body 3 may have a substantially cylindrical shape with preferably round cross-section. The reference number 5 indicates a handle, from which a connection cable 7 may extend, connecting the ultrasound probe 1 with an ultrasound machine 9 (Fig.2) shown schematically.

Characteristically, the elongated body 3 of the ultrasound probe 1 comprises a convex outer surface (substantially round cylindrical in the illustrated embodiment), on which ultrasound sensors 11, also referred to as transducers, are arranged. As shown in Fig. 1A, the ultrasound sensors 11 are arranged according to a two-dimensional matrix of rows and columns. More in particular, the ultrasound sensors 11 in the illustrated embodiment are arranged according to lines parallel to the longitudinal axis A-A of the elongated body 3 of the ultrasound probe 1, the lines being arranged around the longitudinal axis A-A of the ultrasound probe 1, preferably spaced by a constant angular pitch. Each row has preferably equidistant sensors. Moreover, the ultrasound sensors 11 are arranged according to curved lines, in the specific illustrated example according to arcs of circumference, as the elongated body 3 has cylindrical shape with round cross-section. The arcs of circumference follow, and are adjacent to, one another in the axial direction (axis A-A) of the body 3 of the ultrasound probe 1. In practice, the ultrasound sensors 11 are arranged according to straight lines represented by the intersection of the cylindrical outer surface of the elongated body 3 of the ultrasound probe 1 with a plurality of planes belonging to a bundle of planes containing the axis A-A. The curved lines, along which the ultrasound sensors 11 are arranged, represent intersection lines between the outer cylindrical surface of the elongated body 3 and a plurality of parallel planes orthogonal to the axis A-A of the elongated body 3 of the ultrasound probe 1.

Therefore, the matrix of ultrasound sensors 11 (each of which configures an element for emitting and receiving ultrasound waves) is substantially constituted by a series of linear arrays of sensors. The linear arrays extend parallel to one another and to the longitudinal axis A-A of the elongated body 3 of the ultrasound probe 1. Moreover, the ultrasound sensors 11 also define arrays of sensors shaped like an arc of circumference, which extend parallel to one another and each of which lies in a plane orthogonal to the longitudinal axis A-A of the elongated body 3 of the ultrasound probe 1.

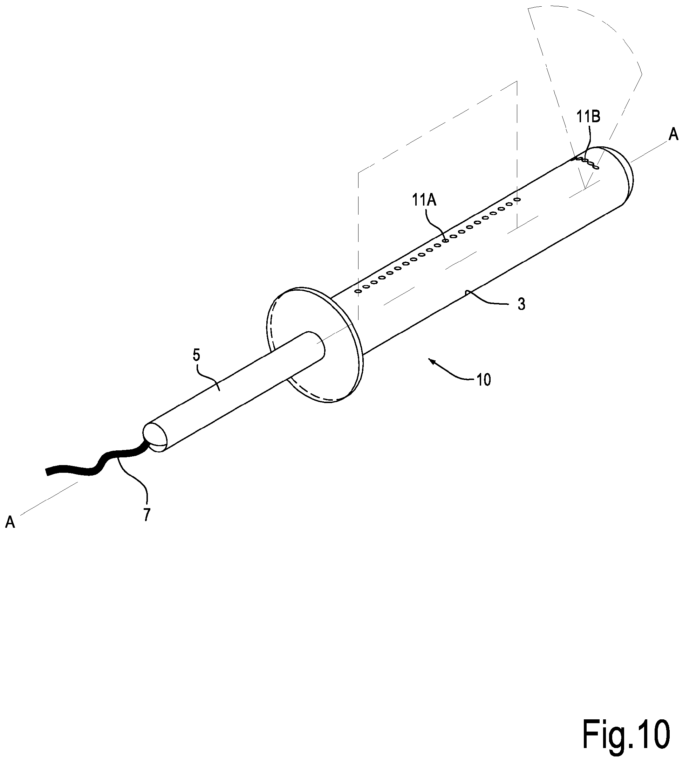

Through the ultrasound machine 9 (Fig. 2), to which the ultrasound probe 1 is connected, it is possible to control the selective and timed actuation of some ultrasound sensors 11 and, more precisely, of single or multiple arrays of ultrasound sensors 11. More in particular, it is possible selectively to actuate the sensors of a linear array or of a group of longitudinal linear arrays, i.e. of arrays constituted by sensors lying on planes containing the longitudinal axis A-A of the elongated body 3 of the ultrasound probe 1. These arrays of sensors allow collecting data for constructing a two-dimensional ultrasound image corresponding to a section of the prostate according to the plane containing the longitudinal axis A-A and passing through the sensors of the array. If more adjacent linear arrays are actuated, a plane containing the probe longitudinal axis passes through each linear array. By sequentially actuating the sensors of single arrays or of the various groups of adjacent longitudinal arrays, parallel to the longitudinal axis A-A of the ultrasound probe 1, a plurality of images is obtained, equivalent to those that can be obtained by rotating a biplane endorectal ultrasound probe around the axis thereof, the probe being of the type shown in Fig. 10 and the use whereof will be explained below. The biplane probe is provided with a single array of sensors that is parallel to the probe longitudinal axis, in addition to an array of sensors transverse to the axis.

Fig. 1A shows two planes P1 and P2 corresponding to two distinct ultrasound images that can be obtained by actuating two arrays or groups of longitudinal linear arrays of ultrasound sensors 11. Reference f1 indicates the rotation direction of the scan plane corresponding to the sequential actuation of longitudinal linear arrays (parallel to the longitudinal axis A-A) of ultrasound sensors 11.

Vice versa, it is possible selectively to actuate the sensors of one or more curved linear arrays, arranged according to an arc of circumference around the longitudinal axis A-A of the ultrasound probe 1. This array or groups of curved linear arrays of sensors allows collecting data for constructing an ultrasound image corresponding to a prostate section according to the central plane of the group on which the sensors of the actuated curved arrays are arranged. By sequentially actuating the sensors of the various curved arrays on circumferential lines adjacent to, and following, one another along the longitudinal axis A-A of the ultrasound probe 1, a plurality of images is obtained, equivalent to those that can be obtained by an ultrasound probe 1 provided with a single round array of sensors through a translation movement parallel to the probe longitudinal axis A-A. To have higher lateral resolutions in a portion of the three-dimensional space of the three-dimensional images where the lesion, the focal tumor, is located, sub-matrices of transducers, i.e. of ultrasound sensors, can be used, belonging to groups of curved and straight linear arrays, as better explained below.

Fig. 1B show three planes P3, P4, P5, following one another in the axial direction f2, wherein the respective ultrasound sensors 11 can be activated sequentially.

In practice, it is possible to capture both a sequence of images according to planes P1, P2 and a sequence of images according to planes P3, P4, P5

It is also possible to actuate ultrasound sensors 11 arranged according to diagonals of the matrix of sensors. In this way, data are acquired for two-dimensional images in oblique planes, as schematically illustrated by the plane P6 in Fig. 1C.

By combining electronic rotation (Fig. 1A) and sliding (Fig. 1B) it is possible to acquire ultrasound data for three-dimensional images. By processing the acquired ultrasound volume data it is possible to have stratigraphic three-dimensional images of sub-volumes of the investigated volume.

It is clearly apparent that, by selectively and sequentially activating arrays of sensors, it is possible very quickly to capture a plurality of two-dimensional images according to a plurality of planes and, more in particular: a plurality of planes containing the longitudinal axis A-A whose number is equal to the number of longitudinal arrays or groups of longitudinal arrays of ultrasound sensors 11; and a plurality of planes orthogonal to the longitudinal axis A-A, whose number is equal to the number of curved arrays or groups of curved arrays of ultrasound sensors 11 following one another in the direction of the longitudinal axis A-A. Time scanning of the various planes can be very fast, and therefore the whole set of data for constructing a three-dimensional image is acquired in a time interval that is much shorter than the time interval required when prior art probes are used.

Moreover, as the passage from a scan plane to the other, both in direction f1 and in direction f2, is electronically controlled, the image quality is significantly higher than that obtained by manually moving the ultrasound probe in direction f1 and/or in direction f2. The scanning pitch in both directions is electronically controlled and corresponds to the pitch according to which the (longitudinal) straight linear arrays and the curved linear arrays are arranged. The spatial distance between the scan planes, along which the single two-dimensional images are obtained, is therefore constant and can be selected, and this improves the quality and reliability of the final three-dimensional image. It should be noted that, when processing data obtained from the two types of images or from diagonal arrays, it is possible to interpolate ultrasound data for obtaining other VOXELs (i.e. elementary portions of volume of 3D images corresponding to the PIXELs for the 2D images) for the stratigraphic images or anyway in any planes, if necessary also images on curved surfaces contained within the volume under investigation.

Thanks to the real time acquisition of the various ultrasound images, and to the high acquisition rate, the navigation is significantly less affected by the patient's movement, even if during interventions the patient is often immovable (anesthetized); in many cases, the navigation is completely not affected by the patient's movement, even for moving parts, like the heart or other organs, moving due to the effect of pulsing flowing blood in arteries or due to breathing.

As mentioned above, the ultrasound images captured during the surgical intervention are merged with magnetic resonance images acquired during separate medical sessions before the intervention. In order for the two types of image to result in a piece of information useful for the surgeon, it is necessary that the images can be superimposed. By using images obtained through the ultrasound probe 1 with electronically controlled array of transducers, described herein, it is possible to have particular advantages as regards the merging of images. Firstly, it is not necessary, in general, to repeat the magnetic resonance images for repeating merging every time the patient moves, thanks to the high acquisition rate of images through electronic scanning, i.e. through selective and sequential activation of the single arrays of sensors 11 adjacent to one another.

Moreover, the quality of both two-dimensional and three-dimensional images, in terms of spatial resolution and signal-to-noise ratio, is higher, as it is possible to use at the same time adjacent groups of ultrasound sensors 11 in different planes, allowing a compound scanning with very high repetition rate for reduced volume, for example where the lesion to be treated is located. In particular, compound scanning performed with sub-matrices of elements in various positions on the cylindrical matrix may improve the side resolution of images, as details are analyzed from different angles.

In view of the description above, it is clearly apparent that with the ultrasound probe 1 structured with a matrix of ultrasound sensors 11 arranged on a curved surface 3 it is possible to have ultrasound images shaped like a circular sector in the transverse plane (see Fig. 1B), and shaped like a rectangle in planes containing the longitudinal axis A-A of the ultrasound probe 1 (Fig. 1A). In order to collect the ultrasound data in an anatomic volume shaped like a cross-section part of a cylinder with round base, an electronic switching is used for sequentially selecting arrays of transducers aligned along a direction parallel to the longitudinal axis A-A, instead of making a rotation by a given angle around the longitudinal axis, which would be necessary for obtaining the rectangular image in a given plane with a prior art ultrasound probe.

Analogously, the images shaped like a sector are obtained, through the ultrasound probe 1, by selecting, in succession, the various arrays of transducers arranged according to a circular arc.

Therefore, instead of the rotation and translation movements necessary when using prior art endorectal ultrasound probes, an electronic switching of ultrasound sensors 11 is provided, keeping the ultrasound probe 1 fixed and always in the same position inside the patient's body, unless adjustment are necessary completely to change the field of view. For increasing the resolution of the signal-to-noise ratio, in order to improve the quality of the ultrasound images, groups of transducers can be used, arranged in areas instead that in arrays; through electronic controls it is possible to perform a compound scanning, adapted to give the operator more useful signals both in diagnostic and treatment phase.

The electronic scanning probe of the type described herein may be manually inserted and kept into position by the sonographer, without the need for performing complex maneuvering movements with the probe to capture the various images, as these movements are replaced by an electronic scanning, as described above.

However, further to improve the sonographer activity, an equipment of the type shown in Fig. 2 can be used. The equipment may be used also to simplify the use of biplane probes, as described below.

The equipment comprises, for example, a gynecology chair or gynecology bed 21, where the patient P rests. The chair 21 interfaces a carriage 23 that may be provided with a slide or a set of slides 25, to which the ultrasound probe 1 may be applied. The carriage 23 may be fastened to the chair 21 and the ultrasound probe can be positioned and inserted into the patient's body, for example by maneuvering it using the set of slides 25. The equipment comprises the ultrasound machine 9, to which the ultrasound probe 1 is connected via the cables 7. Once the probe has been inserted, the operator, using a suitable interface 29, can capture the ultrasound images that can be merged in the ultrasound machine 9 with previously captured and stored magnetic resonance images. The combined image may be shown on a monitor 29 of the ultrasound machine 9; through the image the operator is guided during the intervention on the patient through known minimally invasive surgical instruments selected according to the type of intervention. For example, the intervention may comprise diagnostic biopsies, treatment of prostatic hyperplasia through removal of tissues, ablation of focal tumor lesions through hyperthermia, application of radioactive seeds (brachytherapy), or the like.

Instead of a slide or a set of slides, any other static, possibly adjustable support may be used, having only the function of keeping the ultrasound probe 1 in the right position with respect to the patient, thus avoiding the need for the sonographer to manually support the probe. As the ultrasound probe 1 is an electronic scanning probe, it does not require to be moved while capturing the images and it can have, therefore a very simple support. For example, an articulated arm can be used, or a semi-rigid flexible arm, that can be positioned only once with respect to the patient at the beginning of the intervention.

The real-time ultrasound image allows guiding the operator in the step of inserting one or more needles, cannulas or other instruments via transperineal approach, for instance.

More in particular, according to an embodiment, the following preparatory steps may be performed for collecting ultrasound data for constructing three-dimensional images and ecotomography in planes selected at will.

Firstly, the carriage 23, housing the support and handling structure 25 of the endorectal ultrasound probe, is coupled to the gynecology chair 21.

By means of suitable regulation knobs, the doctor manually aligns the ultrasound probe so that it is ready to be inserted into the patient. To this end, the set 25 may have the following freedom degrees: adjustment of the height from the ground; horizontal translation transversally to the edge of the gynecology chair 21, with which the carriage 23 is associated; adjustment of the inclination of the ultrasound probe 1 around a vertical axis and a horizontal axis, to align the longitudinal axis A-A of the ultrasound probe 1 with the axis of the rectum of the patient P; adjustment of the distance between the end 1A (Fig. 1) of the ultrasound probe 1 from the anus of the patient P. The patient is anesthetized and for keeping him in position on the gynecology chair 21 a small pillow or bed C can be used, that is filled with resin, takes the shape of the patient and solidifies forming a seat for receiving the patient P and keeping him in position.

Once the ultrasound probe 1 has been manually positioned and aligned with the anus of the patient P, the ultrasound probe is inserted into the rectum of the patient P. After having inserted the ultrasound probe 1, it can be finely positioned by acquiring data to verify, from the resulted ultrasound images, the right positioning so as to bring the anatomical portion of interest for the subsequent intervention within the field that can be represented with the three-dimensional ultrasound images.

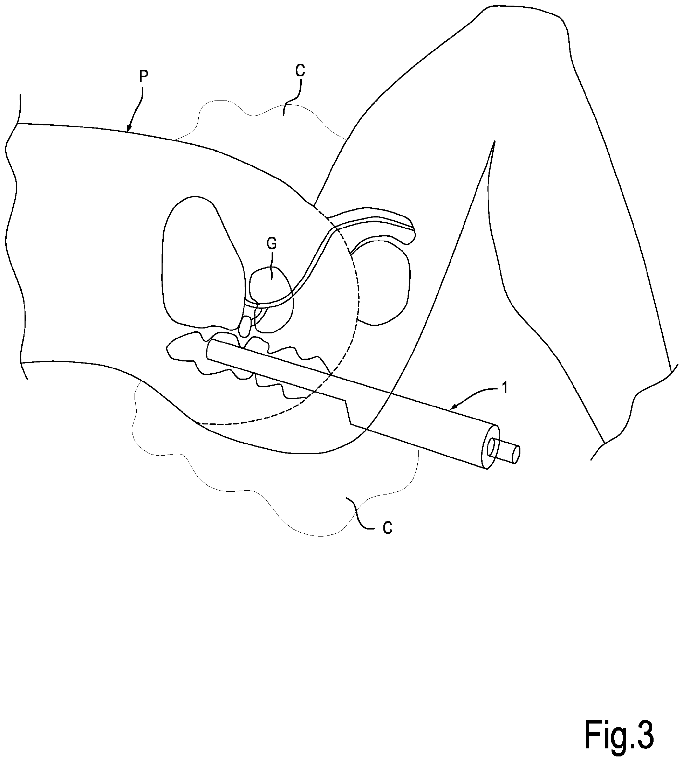

Once the ultrasound probe 1 has been correctly positioned into the patient's rectum, as schematically shown in Fig. 3, it can be activated to perform electronic scanning in direction f1 and/or f2 (Figs. 1A and 1B) and to acquire data for constructing three-dimensional images of the prostate gland G (Fig. 3).

If a biplane probe is used, as described below, the support may comprise a system of slides or other system for displacing the probe. These systems are used manually or electrically controlled to select the images in the planes chosen by the doctor through the physical movement of the probe, as the latter is not provided for the electronic scanning of the ultrasound sensors 11.

In the illustrated embodiment, an articulated arm is provided, connecting the probe-holding carriage 23 to the chair with related moving mechanisms for positioning the probe correctly with respect to the patient's body; in other simpler embodiments the electronic probe 1 may be held manually by the operator and inserted into the patient's rectum, to remain in this position, where the above-mention electronic scanning is performed through selective activation of the single ultrasound sensors of the matrix.

With the electronic probe it is possible to assume that there are no relative movements between patient and probe, as the sequence of the scan planes is obtained while the probe is maintained stationary, by electronically switching the ultrasound sensors i.e. the ultrasound beam. However, the described equipment can be also used in treatments for reducing the benign prostatic hyperplasia with minimally invasive methods. In this case, as the patient is awake, the presence of sensors for the six degrees of freedom on the endorectal ultrasound probe and the outer ultrasound probe may be useful to balance relative patient-probe movements. In particular, this could be useful in the case where it is necessary, having a precise knowledge of the three-dimensional situation of the whole organ, to move the needles or other instruments supplying energy inside the prostate gland.

In some embodiments, in addition to the anatomical images captured through the ultrasound probe 1, data can be also acquired and merged relating to anatomical conspicuous points, i.e. anatomical details selected as reference points, in order more precisely to combine, or merging, the three-dimensional image obtained through the ultrasound probe 1 and high-resolution three-dimensional anatomical images obtained during a previous session through a different imaging equipment, for example nuclear magnetic resonance, computerized axial tomography, positron tomography, or other high-resolution imaging technique.

It should be noted that, when capturing images of the area of interest (the prostate, in the example illustrated herein) through magnetic resonance or other imaging techniques not using an ultrasound probe, the patient's organs are not subjected to the compression that, vice versa, occur when capturing ultrasound images through an endo-rectal ultrasound probe 1, due to the presence of said probe in the patient's body. The probe occupies a volume that otherwise would be free or virtual, and causes therefore a compression of the adjacent soft tissues and a subsequent deformation of the organs under investigation. The same situation occurs every time the ultrasound image is captured through an endocavity probe inserted into a cavity associated with the organ under investigation.

In the specific case of prostate ultrasound, in the images obtained through the ultrasound probe 1 the prostate gland G is deformed due to the presence of the ultrasound probe 1, compressing the surrounding soft tissues, including the prostate gland. This effect makes it difficult to merge the ultrasound images and the images captured through magnetic resonance or other imaging technique, useful for detecting malignant focal lesions that shall be treated accurately due to the small dimension thereof.

In order to overcome or to alleviate this drawback, an improved embodiment of the invention provides for the use of a solid and substantially rigid body, here below referred to as "dummy probe", having outer shape and dimension, as well as mechanical strength, substantially equal to those of the ultrasound probe 1. In this way, when the dummy probe is inserted into the patient's body, in the same position where the ultrasound probe 1 shall be inserted, the dummy probe occupies the same volume occupied by the ultrasound probe 1, therefore inducing in the surrounding soft tissues the same compressive deformation caused by the ultrasound probe 1. The dummy probe is inserted into the patient's body before acquiring images through magnetic resonance or other imaging technique. In this way, the images captured through magnetic resonance are captured on tissues subjected to the same deformation to which they will be subjected when the ultrasound probe 1 will be inserted into the patient's body.

The material, of which the dummy probe is made, shall not disturb the acquisition, by the magnetic resonance machine or other imaging machine, of data related to the chemical physical features of the patient's biological tissues under investigation. Moreover, the material, of which the dummy probe is made, is preferably a bio-compatible plastic material, either disposable or re-sterilizable.

By using a dummy probe the anatomical images obtained through the two techniques (ultrasound and magnetic resonance, for example) are perfectly comparable during the step of merging the two types of images for navigating during the minimally invasive surgical intervention.

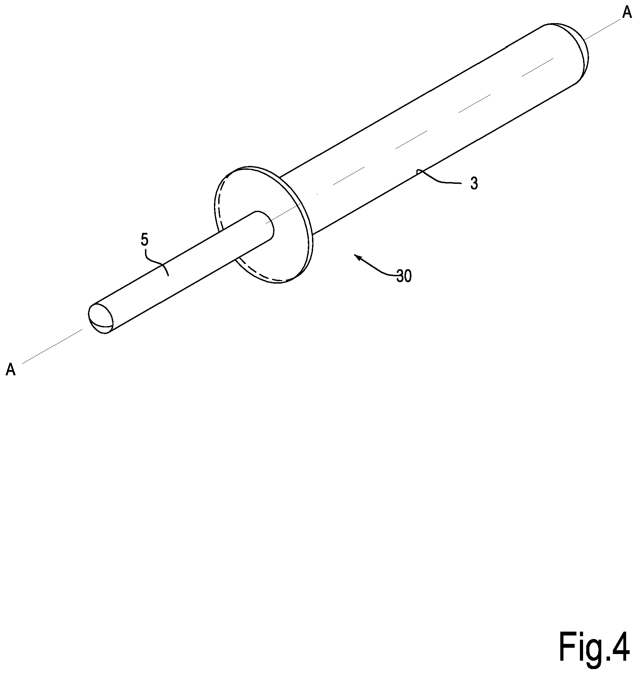

Fig. 4 schematically shows an axonometric view of a dummy probe 30, morphologically equal to the ultrasound probe 1, but devoid of ultrasound sensors 11 and of connection cables 7.

In some embodiments, it is possible to use the profile of the impression in the tissues due to the presence of the dummy probe 30 as conspicuous structure, i.e. as a reference structure, to align the high-resolution diagnostic images obtained through magnetic resonance, or other imaging technique, with the real-time, less defined images obtained by the ultrasound probe 1. The compressed tissues can be seen in any two-dimensional image obtained by cutting out the whole captured three-dimensional image, and therefore the superimposition of ultrasound images with high-resolution images is possible in any plan of view.

In some embodiments, the dummy probe 30 may be entirely or partly made of materials that can be detected in high-definition images captured through magnetic resonance or other suitable technique. For example, it is possible to produce structures or simple reference points inside the dummy probe 30 made of materials visible in magnetic resonance images.

If the materials used for realizing producing points inside the dummy probe disturb, due to the absorption features or other features thereof, the images obtained with one or the other or both the imaging techniques, the dummy probe 30 may be made hollow and liquid-tight, at least in the part destined to be inserted into the patient's body. Once the images have been captured through magnetic resonance, or other high-resolution imaging technique, without removing the dummy probe from the patient's body, a structure comprising conspicuous points is inserted in the empty volume inside of the dummy probe, and a further image is captured, to obtain the magnetic resonance anatomical images where the conspicuous points are visible. The two magnetic resonance images are then merged to obtain a compound magnetic resonance image that, in this way, keeps the evidence and the localization of the lesion to be treated together with the representation of the inserted probe, with the conspicuous structures thereinside. This whole magnetic resonance image of the anatomical part in question comprises: the geometrical anatomical deformations due to the presence of the dummy probe 30; the visible profile of the dummy probe 30 and therefore also the interface between probe and compressed tissues touching the probe; any conspicuous or reference points; the lesion to be treated, that could be displaced due to the presence of the ultrasound probe. The image is used for merging, without systematic errors due to the presence of the endorectal ultrasound probe 1, the magnetic resonance image and the ultrasound image used during treatment.

In some embodiments, for correctly superimposing the high-resolution magnetic resonance images or other high-resolution images on, i.e. for merging them with, the ultrasound image obtained in real-time through the ultrasound probe 1, it is possible to use, as reference points, the area of compressed tissues surrounding the probe. These tissues are visible both in the magnetic resonance image and in the ultrasound image generated in real-time through the ultrasound probe 1. In addition to, or alternatively, the images of anatomical structures that can be easily identified in both types of image, for example bone structures, can be used as conspicuous points, i.e. as reference points.

In other embodiments, to have a higher accuracy in superimposing the high-resolution images on, and merging them with, the ultrasound images, it is possible to use conspicuous points, i.e. reference points provided on the surface of the dummy probe 30 and of the ultrasound probe 1, or inside them. In this regards, it should be noted, however, that the ultrasound probe 1 generates a three-dimensional ultrasound image of the area of interest, where the probe is not visible, as the ultrasound sensors 11 are provided on the outer surface of the ultrasound probe 1. Therefore, in order to use conspicuous or reference points provided on the surface of the probe or inside it, it is necessary to capture further ultrasound images through an outer ultrasound probe, for example an outer probe applied to the pubic zone or the perineal zone or both. In Fig. 2 an outer ultrasound probe is schematically indicated with reference number 2. These further ultrasound images are captured after having previously inserted the dummy probe 30 or the ultrasound probe 1 into the patient's body. For example, the ultrasound images captured through the outer ultrasound probe may be captured during the same session when the magnetic resonance images are acquired. In this case, usually the dummy probe 30 has been inserted into the patient's body.

In other embodiments, the ultrasound images captured through the outer ultrasound probe are acquired during the treatment, when the endorectal ultrasound probe 1 has been inserted into the patient's body. If dummy probe 30 and ultrasound probe 1 have the same shape and dimension, as well as the same conspicuous or reference points, it is theoretically possible to proceed in both ways.

By merging the ultrasound images captured through the endorectal ultrasound probe 1 with the ultrasound images captured through the outer ultrasound probe, ultrasound images are obtained, where the conspicuous points represented by the ultrasound probe 1 are visible.

In some embodiments, both the endorectal ultrasound probe 1 and the outer ultrasound probe are provided with sensors (for instance infrared or electromagnetic sensors, of known type) to detect the spatial position of each probe in the six degrees of the freedom (three translation axes, three rotation axes). This allows merging the ultrasound images obtained through the two probes in easier manner.

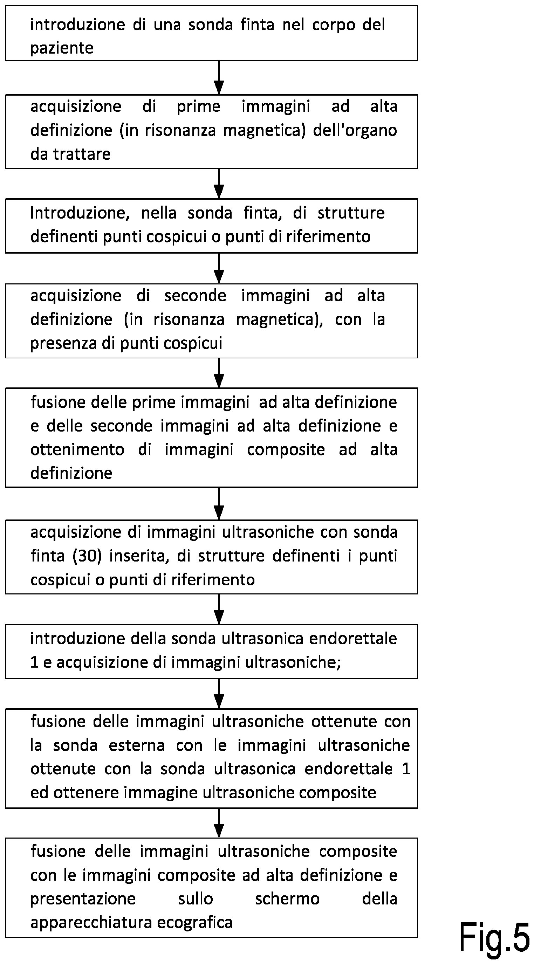

The sequential steps of a minimally invasive surgical intervention on malignant focal lesions in the prostate, using a dummy probe 30 and an endo-rectal ultrasound probe 1, if necessary in combination with an outer ultrasound probe, will be described in greater detail below. The intervention provides for merging magnetic resonance images (or other high-resolution images), for diagnosis and localization of the lesion to be treated), and electronically scanned three-dimensional ultrasound images obtained with the ultrasound probe 1 described above. The steps described below are summarized in the flow chart of Fig. 5.

The first step is a session for acquiring high resolution images of the organ (the prostate in the illustrated example) to be investigated and treated, for example through nuclear magnetic resonance, positron tomography, computerized axial tomography, or in general through a high-definition imaging technique allowing diagnosis and localization of the lesion in the organ, in the present case the prostate.

Before acquiring the high-definition images, a dummy probe 30 is inserted into the rectum of the patient P, the probe having shape and dimension equal to those of the ultrasound probe 1 to be used in the treatment step.

As mentioned above, the images may be captured firstly using a hollow dummy probe 30; then, the image acquisition is repeated after having inserted, inside the dummy probe 30, structures defining reference (conspicuous) points that can be detected through the technique used for capturing the anatomical images. The diagram of Fig. 5 provides for this double step. If two distinct steps of image acquisition are performed, i.e. a first step for capturing images without conspicuous points and, later, a second step for capturing images with conspicuous points, the two images obtained in the first and in the second step are merged to have images of the anatomical district under investigation that are devoid of artifacts that could be caused by the structures defining the reference points. In practice, the obtained images contain conspicuous points, i.e. reference points, but do not contain any perturbation due to the presence of the structures generating the same reference points.

If the structure defining the conspicuous points do not cause artifacts in the high-resolution images, it is possible to capture only one series of images with the dummy probe 30 provided with the structure defining the conspicuous points, without the need for repeating the image acquisition twice, i.e. with and without the structure defining the conspicuous points.

The obtained high-resolution images contain, in addition to the conspicuous points, also the profile of the dummy probe 30 that can be easily detected in the high-resolution image.

Then, for performing the surgical intervention on the patient, through the ultrasound probe 1, ultrasound images are captured that are merged with the high-definition images previously acquired through magnetic resonance or other high-definition imaging technique. If, as mentioned above, merging is performed using, as reference or conspicuous points, only patient's tissue structures, for example bones visible in one or the other of the various captured images, then only the ultrasound images shall be captured through the ultrasound probe 1, that are then merged with the high-definition images. The operator can view the image resulting from merging on the monitor 29 of the ultrasound machine 9, or on a dedicated monitor.

For merging the real-time ultrasound images with the high-definition images it is possible to use, in addition to patient's biological structures (in particular hard tissues such as bones), also the profile of the probe identified by the surface of the tissues surrounding the probe, which define the interface between tissues and probe and therefore allow visualizing the volume occupied by the probe.

If, for a more accurate merging, conspicuous points defined on the two types of images by structures housed in the dummy probe 30 or there around, are desired, the image of these points shall be captured by means of an outer ultrasound probe 2, for instance a suprapubic probe. This is the case in the flow chart of Fig. 5.

The ultrasound images captured by means of the outer ultrasound probe 2, which contain the structures defining the conspicuous points inside the probe or on the surface thereof, are merged with the ultrasound images captured by the endorectal ultrasound probe 1, so as to have a complete ultrasound image of conspicuous points on the surface of, or inside, the probe. The conspicuous or reference points are present also in the magnetic resonance image; therefore the subsequent merging of the ultrasound image containing conspicuous points with the high-resolution image containing conspicuous points is more accurate and easier.

As mentioned, for aligning the images, thus facilitating the merging of the various images, it is possible to use also the pubic bone, the sacrum, the coccyx, the prostate or other biological structures in different longitudinal and transverse cross-sections, in addition to the reference or conspicuous points artificially provided on, or in, the dummy probe 30, or, alternatively, provided directly on the endorectal ultrasound probe 1. Alternatively, or in addition, the use of infrared or electromagnetic sensors stably arranged on the ultrasound probes and on the patient's body can give further elements for merging the images.

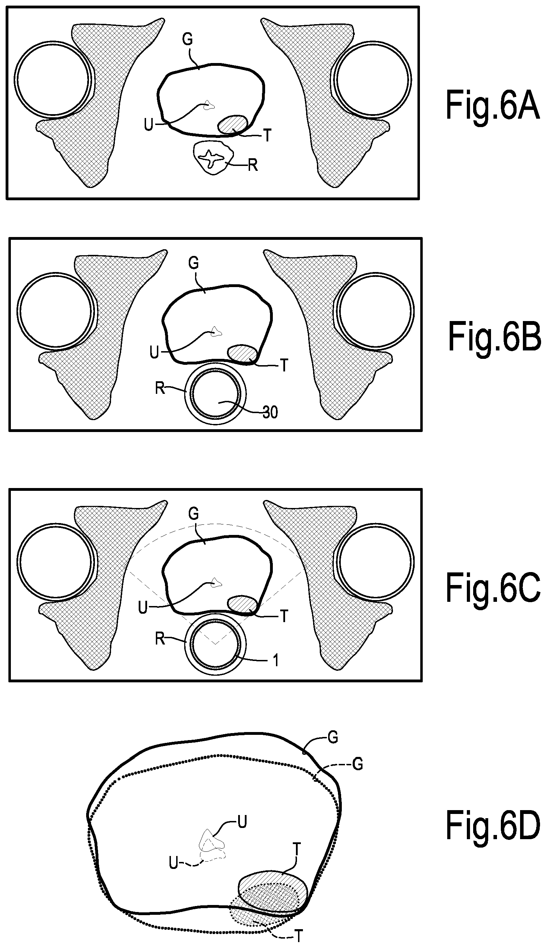

The method described above and summarized in the diagram of Fig. 5 is further illustrated in Figs. 6A-6D. Fig. 6A schematically shows an axial image of a patient's pelvic region, acquired through magnetic resonance. The letter G indicates the prostate gland, U indicates the urethra, R indicates the rectum and T indicates a tumor lesion. The image of Fig. 6A has been obtained without a dummy probe in the rectum R.

Fig. 6B schematically represents the same image of Fig. 6A, but, in this case, obtained through the insertion of a dummy probe 30 into the patient's rectum R. It should be noted that the presence of the dummy probe 30 causes a compression of the organs surrounding the rectum cavity where the dummy probe 30 has been inserted and, consequently, a displacement of these organs.

Fig. 6C schematically shows an ultrasound image obtained with an ultrasound probe 1, having the same shape and dimension as the dummy probe 30, inserted into the rectum R. It should be noted that, due to the presence of the ultrasound probe 1, the surrounding tissues have been subjected to a compression substantially equal to the compression caused by the dummy probe 30 shown in Fig. 6B.

Fig. 6D shows the two images of Fig. 6A and of Fig. 6B superimposed on each other, to show the effect of the displacement of the organs surrounding the cavity where the dummy probe 30 is inserted while capturing the magnetic resonance images. If, instead of the image of Fig. 6B, the image of Fig. 6A is merged with the image of Fig. 6C, two inconsistent images would be superimposed with each other.

The use of the dummy probe 30 prevents this drawback and allows merging images obtained through a high-definition imaging technique (imaging diagnostics), such as magnetic resonance or other techniques mentioned above, with ultrasound images captured through the endocavity probe 3. The two images have been captured by distinct equipment, and not with the same endocavity probe; this allows to optimize the acquisition of both the first, high-resolution image and the second, ultrasound image.

What described above with reference to a single image also applies when acquiring a plurality of two-dimensional images, for example on different planes, to obtain three-dimensional images of the organ under investigation.

The first image(s), obtained through the first imaging technique, for example magnetic resonance, and the second image(s), obtained through ultrasound probe, may be captured in different medical sessions, also separated by a period of time. Alternatively, the two images or series of images may be captured in the course of the same medical session.

For example, in a session it is possible to acquire, through a magnetic resonance machine or other high-definition imaging machine, the first image(s) using the dummy probe 30 housed in the patient's cavity (the rectum in the example of prostate investigation). The stored images are subsequently used during a session for acquiring ultrasound images. The ultrasound images can be used in real time. The ultrasound machine may be programmed so as to superimpose, on the monitor, the real-time ultrasound images, captured though the endocavity probe 1, with the first image(s) captured through the first imaging technique.

With the described technique, interferences are avoided between the endocavity ultrasound probe and the operation of the high-definition imaging machine, for example the magnetic resonance machine.

The method described above using the dummy probe during the step of acquiring images through high-definition technologies can be used also with prior art biplane probes, instead of electronic scanning probes as described above. The biplane probe requires manual or servo-assisted rotation and translation movements to be preferably performed by means of equipment described below and illustrated in the drawing.

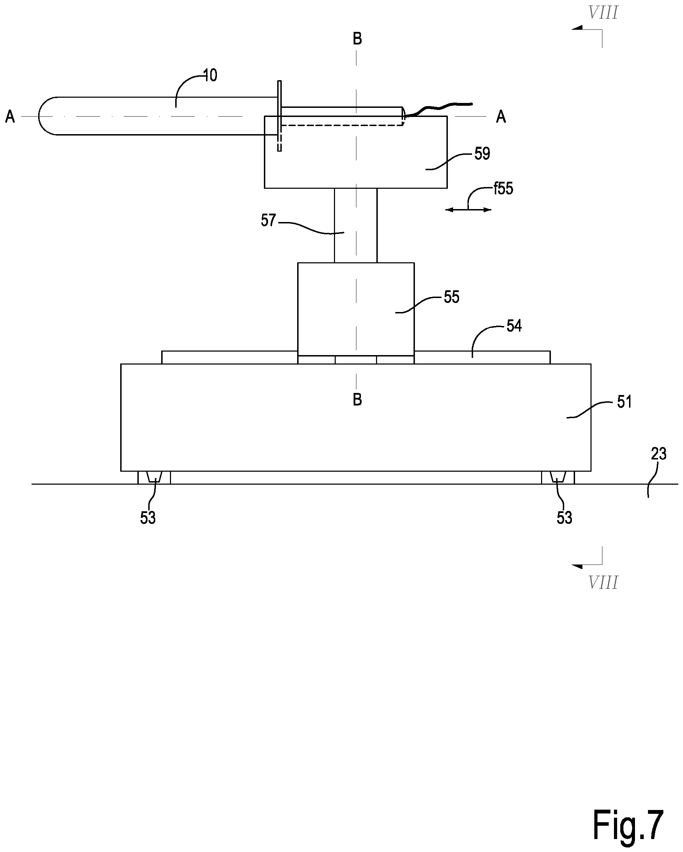

Figs. 7 to 10 show a biplane endocavity ultrasound probe 10 usable for implementing a procedure of the type described above. The probe 10 has an elongated body 3 having a longitudinal axis A-A. The elongated body 3 may advantageously have a substantially cylindrical shape with preferably round cross-section. The reference number 5 indicates a handle, from which a connection cable 7 can extend, connecting the ultrasound probe 1 with a schematically represented ultrasound machine 9 (Fig. 1).

Characteristically, the elongated body 3 of the ultrasound probe 10 comprises a convex outer surface (substantially round cylindrical in the illustrated embodiment), on which ultrasound sensors 11A, 11B are arranged. As shown in Fig. 10, the ultrasound sensors 11A are arranged according to a straight linear array parallel to the axis A-A, whilst the ultrasound sensors 11B are arranged according to a curved linear array provided on a plane orthogonal to the axis A-A of the probe 10.

Through the ultrasound machine 9 (Fig. 1), to which the ultrasound probe 10 is connected, it is possible to control the selective and timed actuation of some ultrasound sensors and, more precisely, of one or the other or both the arrays of sensors 11A, 11B. More in particular, it is possible to actuate selectively the ultrasound sensors 11A of the longitudinal linear array, i.e. the array parallel to the longitudinal axis A-A of the elongated body 3 of the ultrasound probe 1. This array of sensors allows collecting data for constructing a two-dimensional ultrasound image corresponding to a section of the prostate according to the plane containing the longitudinal axis A-A and passing through the sensors of the respective actuated array. In the case of the biplane probe 10, only one longitudinal array of sensors 11A and only one transverse array of sensors 11B are provided. Therefore, only by rotating the biplane ultrasound probe 10 around the longitudinal axis A-A thereof and actuating the sensors 11A in each angular position taken by the ultrasound probe 10, a plurality of two-dimensional images is obtained that, merged together, result in a three-dimensional image of a portion of the organ under investigation.

Analogously, by activating the ultrasound sensors 11B of the curved array of the biplane probe 10, and moving the biplane probe 10, while inserted in the patient's rectum, parallel to the longitudinal axis A-A thereof, a plurality of transverse images is obtained, that can be merged to have a three-dimensional image.

For the biplane probe 10, the images that can be captured by activating the ultrasound sensors 11A, 11B can be merged together manually in a known manner. However, in order to have a more accurate result, a particular equipment is provided, described in greater detail with reference to Figs. 2 to 9, to control in a more accurate way the movement of the ultrasound probe 10 after it has been inserted into the patient's body.

The equipment using the biplane ultrasound probe 10 may be configured as illustrated in Figs. 2 to 4, and can be used with an ultrasound machine of the type shown in Fig. 1. It should be noted that this equipment can be also used to support an electronic scanning ultrasound probe 1 as described above. In this case, the probe movement members can remain inactive, as the scanning is electronically controlled.

The equipment for using the biplane probe 10 comprises, for instance, the gynecology chair 21, on which the patient P rests, as mentioned above. To the chair 21 the arm or carriage 23 is connected, which may be provided with a set of slides 25, described in greater detail below with reference to Figs. 7 to 9, on which the ultrasound probe 10 may be applied, in order to move the biplane slide 10 in controlled fashion.

The carriage 23 is fastened to the chair 21, and the ultrasound probe 10 is positioned and inserted into the patient's body P, for example through a manual maneuvering using the set of slides 25.

The equipment further comprises the ultrasound machine 9, to which the biplane ultrasound probe 10 is connected through the cables 7. After having inserted the probe, the operator may capture, through a suitable interface 27, the ultrasound images that can be merged with magnetic resonance images captured in advance and stored in the ultrasound machine 9. The combined image may be displayed on a monitor 29 of the ultrasound machine 9; through the combined image the operator is guided during the intervention on the patient using known minimally invasive surgical instruments selected according to the type of intervention. For example, the intervention may comprise diagnostic biopsies, treatment of prostatic hyperplasia through removal of tissues, ablation of focal tumor lesions through hyperthermia, application of radioactive seeds (brachytherapy) or the like.

The real-time ultrasound image allows guiding the operator in the step, for example, of inserting one or more needles, cannulas or other instruments via transperineal approach.

The set of slides 25 for moving the biplane probe 10 in controlled fashion may comprise a first slide 51, movable along first guides 53 that are integral with the carriage 23. Second guides 54, along which a second slide 55 is movable, are rigidly fastened to the first slide 51. The references f51 and f55 indicate the direction of movement of the slides 51 and 55 along the respective guides 53 and 54. Advantageously, the two directions f51 and f55 are orthogonal to one another.

A column 57 carrying a third slide 59 is mounted on the second slide 55. The third slide 59 rotates according to arrow f59 around a vertical axis B-B, coinciding with the axis of the column 57. Instead of a column, a rotating plate may be provided, rotatable on the second slide 55.

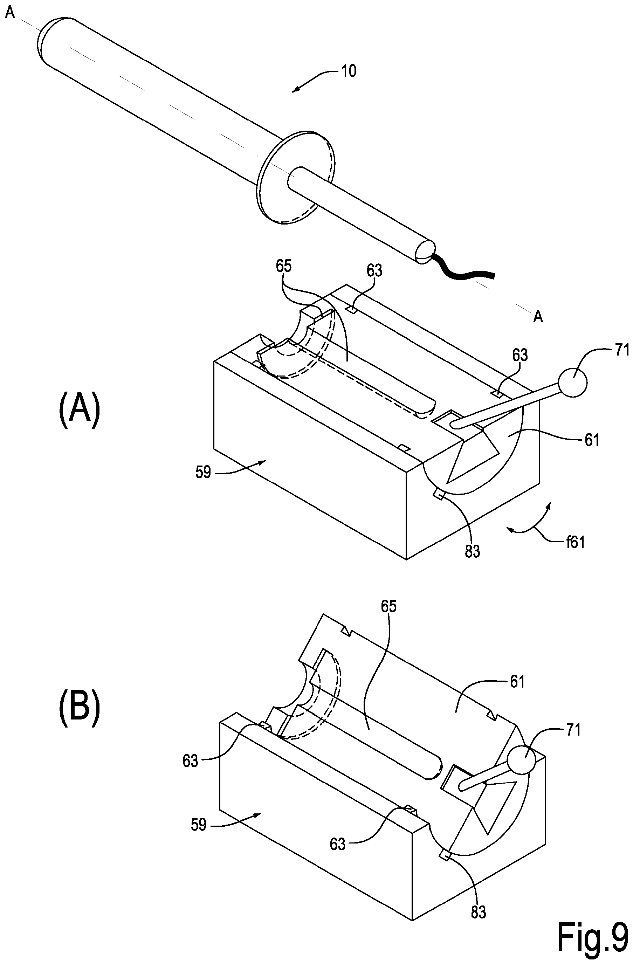

As shown in particular in Figs. 9A and 9B, the third slide 59 comprises a cradle 61, mounted on the third slide 59 so as to be able to rotate around a horizontal axis orthogonal to the axis B-B. The rotation movement of the cradle 61 is represented by the double arrow f61 and is guided through curved guides 63 integral with the slide 59. The rotation axis according to the double arrow f61 is parallel to the translation direction f55.

The cradle 61 has a seat 65 where the ultrasound probe 10 is inserted and blocked. The probe can be fastened on the cradle 61 through blocking members, not shown, so that the ultrasound probe 10 is rigidly constraint to the cradle 61 and cannot move with respect thereto.

The movements of the slides 51, 55, 59 and of the cradle 61 with respect to one another and to the carriage 23 may be controlled by actuators, not shown, for example electronically controlled electric motors. In other embodiments, the movements may be controlled manually, using levers, knobs or other interfaces allowing the operator to move the various slides and the cradle relative to one another in a very accurate and precise fashion. Fig. 9 schematically shows a lever 71 for controlling the rotation movement of the cradle 61 around the horizontal rotation axis. When the ultrasound probe 10 is mounted on the cradle 61, the axis A-A of the ultrasound probe coincides with the rotation axis of the cradle 61 relative to the second slide 59.

Therefore, the set of slides 25 described above allows the sonographer to control the ultrasound probe 10 according to two degrees of translation freedom (arrows f51 and f55) and according to two degrees of rotation freedom (arrows f61 and f59). It is also possible to add a further degree of translation freedom according to a vertical axis and of rotation freedom according to a horizontal axis orthogonal to the axis A-A of the ultrasound probe 1, when this latter in blocked in the rotating cradle 61. These last two degrees of freedom may be useful for initially positioning the ultrasound probe 10 with respect to the patient, but they are not used for acquiring the ultrasound images.

For the biplane probe 10, the movement according to the directions f61 and f55 are useful to move the arrays of ultrasound sensors 11A, 11B when the probe is in the patient's body, to capture two-dimensional images according to two bundles of planes. The rotation according to arrow f61 allows acquiring a plurality of images according to a bundle of planes containing the axis A-A of the ultrasound probe 10, coinciding with the rotation axis of the cradle 61 when the ultrasound probe 10 is housed and blocked in the cradle 61. The various images are captured with the array of ultrasound sensors 11A.

The translation according to the arrow f55 allows acquiring a plurality of images according to a bundle of planes parallel to one another and orthogonal to the axis A-A when the ultrasound probe 10 is made translate by moving the slide 55 along the guides 54. The various images are captured through the array of ultrasound sensors 11B.

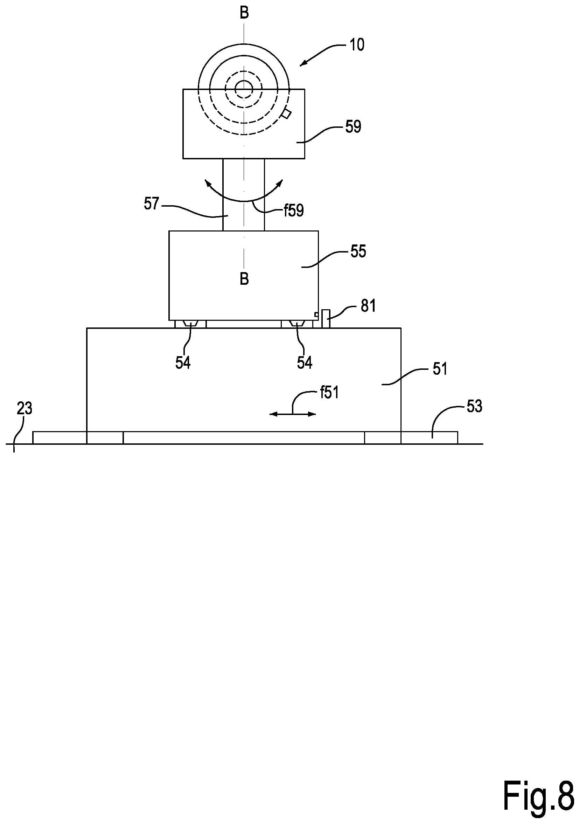

The captured images can be merged together to have a three-dimensional image or a plurality of three-dimensional images of a suitable portion of the organ under investigation, in this example the prostate. To have very accurate three-dimensional images constructed by merging the two-dimensional images, it is necessary to control the movement of the ultrasound probe 10 at least in the movements according to the arrows f55 and f61. To this end, an encoder 81 may be provided (schematically shown in Fig. 8) to detect the movement of the ultrasound probe 10 in the direction f55. The encoder may be any encoder, for example using an optical, magnetic, or mechanical system, or any other system, provided that it is able to detect the movements of the slide 55 in the direction f55, and, if necessary, also the speed of motion. Similarly, an encoder 83 (schematically shown in Figs. 9A and 9B) may detect the movement, and if necessary also the speed, of the cradle 61 when rotating according to the arrow f61.

The signals detected by the encoders may be delivered to a control unit, not shown. In this way, the operator has a tool facilitating the controlled movement of the ultrasound probe 10 along the two degrees of freedom necessary for capturing the two-dimensional images according to the various scan planes necessary for constructing a three-dimensional image. The electronic control by means of the encoders may be used, for example, for controlling the translation and rotation speed, preventing too fast movements, or avoiding too large or too small rotation or translation pitches. For example, the operator can be noticed if the imparted movement is too fast or too slow. Or, in case the movement is imparted in stepped fashion, and at every step the ultrasound probe 10 is stopped for capturing the corresponding two-dimensional image, a control may be provided informing the operator in case the steps imposed to the ultrasound probe 10 are too small or too large.

The data from the encoders may be communicated to the operator through a suitable interface, for example a monitor of the ultrasound machine 9. In addition to the visualization on the monitor, also acoustic signals may be provided, for example to signal the operator that the performed movement is right, or too fast, or too slow, too large or too small, etcetera.

The controls through the encoders 81, 83, together with the movement guided through the guides described above, allow an accurate scanning of the organ under investigation, the prostate in this example.

According to some embodiments, for collecting ultrasound data for constructing three-dimensional images and ecotomography in planes at will, the following preparatory steps may be performed.

Firstly, the arm or carriage 23, housing the support and handling structure 25 of the endorectal ultrasound probe 10, is made integral with the gynecology chair 21.

Through adequate regulation knobs, the doctor manually aligns the ultrasound probe 10 so that it is ready to be inserted into the patient. To this end, the arm or carriage 23 may have the following freedom degrees: adjustment of the height from the ground; horizontal translation transversally to the edge of the gynecology chair 21, with which the arm or carriage 23 is rigidly associated; adjustment of the inclination of the ultrasound probe 10 around a vertical axis and a horizontal axis, to align the longitudinal axis A-A of the ultrasound probe 10 with the axis of the rectum of the patient P; adjustment of the distance between the end 1A (Fig. 10) of the ultrasound probe 10 from the anus of the patient P. The patient is anesthetized and for keeping him in position on the gynecology chair 21 a small pillow or bed C can be used, that is filled with resin, which takes the shape of the patient and solidifies forming a seat for retaining the patient P and keeping him in position.

Once the ultrasound probe 10 has been manually positioned and aligned with the anus of the patient P, the ultrasound probe 10 is inserted into the rectum of the patient P. After having inserted the ultrasound probe 1, it can be finely positioned by acquiring data to verify, from the resulted ultrasound images, the right positioning so as to bring the anatomical portion of interest for the subsequent intervention within the field that can be represented with the three-dimensional ultrasound images.

Once the ultrasound probe 10 has been correctly inserted and positioned in the patient's rectum, as schematically shown in Fig. 5, it can be actuated and moved by the operator according to the arrows f55 and f61, as described above.

In both the described embodiments, endocavity ultrasound probes are used, in the illustrated example in particular, endorectal probes with a particular structure.

In an embodiment, the probe 1 is an electronic scan probe adapted to collect data for constructing three-dimensional ultrasound images in real time. In practice, the probe is structured as a two-dimensional matrix of ultrasound sensors arranged on a curved convex surface adapted to allow the acquisition of both longitudinal and transverse images, i.e., according to a first bundle of planes containing a longitudinal axis of the probe and to a second bundle of parallel planes orthogonal to the longitudinal axis, simultaneously. In this way, the probe does not require mechanical scanning movements, as the ultrasound beam is electronically moved in the space according to the directions necessary for capturing data from an anatomical volume of interest, the data being used for creating three-dimensional images and cross-sections according to the planes necessary for diagnostic purposes or for treatment activities.

In the second case, the probe 10 is a probe with only two arrays of transducers (biplane probe), one longitudinal array and one transverse array, requiring a rotation and translation movement to provide an ordered sequence of two-dimensional images for constructing the three-dimensional image to be merged. In both cases it is possible to capture a plurality of two-dimensional images, that can be merged together to have a three-dimensional image. This can be merged with a three-dimensional image obtained through a high-definition imaging system.

Both probes 1 and 10, but especially the electronic scanning ultrasound probe 1, allow obtaining further advantages.

Specifically, through a suitably timed control of the transducers or ultrasound sensors 11 it is possible to perform a compound scanning to increase the side resolution, and having a stratigraphic view of the organ under investigation.

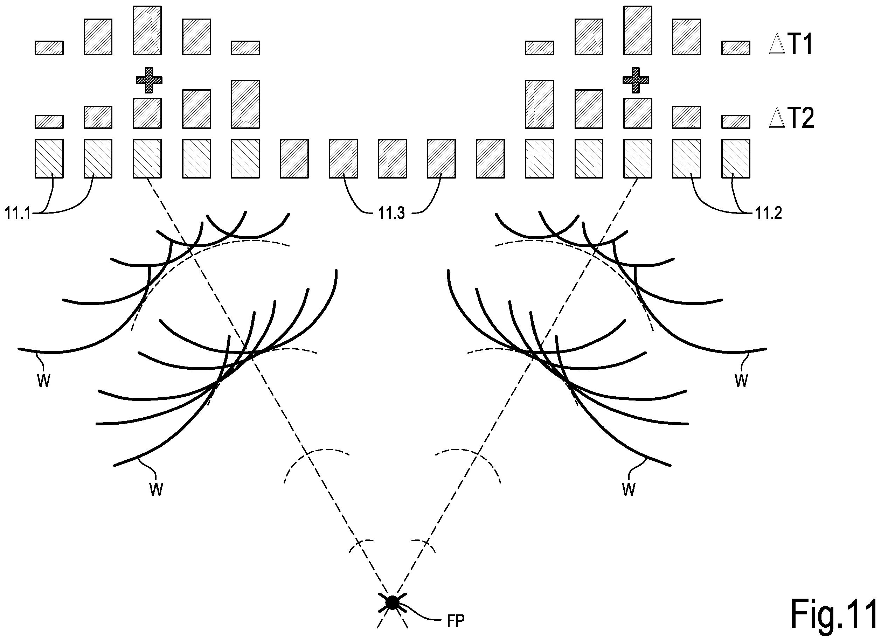

Reference should be made to Fig. 11 for a better understanding of these aspects. In Fig. 11 a single straight linear array of ultrasound sensors is shown, divided into three groups of ultrasound sensors, and more in particular: two groups or clusters of end ultrasound sensors 11.1 and 11.2 and a group or cluster of intermediate sensors 11.3. In the case of the electronic scanning ultrasound probe 1, the linear array illustrated in Fig. 11 may be one of a group of arrays parallel and adjacent to one another, arranged around the probe longitudinal axis A-A. In the plane of Fig. 11 it is shown how the ultrasound sensors or transducers 11.1 and 11.2 are excited through electric pulses to emit ultrasound waves with suitably oriented wavefronts. The electric pulses are suitably delayed. A similar delay is applied during the step of receiving the ultrasound signal reflected by the tissue structures under investigation.

In the upper part of the diagram of Fig. 11 two distinct time delays are shown and indicated with ΔT1 and ΔT2. The delay ΔT1 has a parabolic trend and the delay ΔT2 has a linear trend along the respective portion of array of ultrasound sensors. In practice, on each group of ultrasound sensors 11.1 and 11.2, each transducer, i.e. each ultrasound sensor 11, is excited with a delay, with respect to the adjacent sensors, given by the sum of the respective delays ΔT1 and ΔT2. In this way, inclined wavefronts W are generated, focusing towards a region FP. This allows higher side resolution of the image.

In the case of an electronic scanning probe 1 provided with a two-dimensional matrix of ultrasound sensors 11, this control mode can be used also in tangential direction, i.e. using not only a single array of ultrasound sensors, but a plurality of linear arrays adjacent to one another. On this plurality of linear arrays of ultrasound sensors sub-groups of ultrasound sensors may be identified, each of which is constituted by a sub-matrix of sensors.

Using suitably modulated time delays for exciting the sensors belonging to consecutive linear arrays in tangential direction, it is possible to have a focus in two direction towards a point FP, further increasing the resolution of the obtained ultrasound images.

More in general, for each sub-matrix of ultrasound sensors 11 it is possible to generate wavefronts with variable inclination and, if necessary, focused towards a portion of the organ under investigation. By using at least two sub-matrices of ultrasound sensors 11, in an equivalent manner to that described with reference, in Fig. 11, to portions of array (therefore one-dimensional sub-matrices), a compound view of a portion of the organ under investigation is achieved.

This operating mode has many advantages. For example, it is possible to investigate limited portions of tissue by selecting a sub-set of ultrasound sensors 11 and focusing the ultrasound energy in a small volume of the organ under investigation. The time necessary for scanning a reduced anatomical volume allows high repetition rate and, therefore, a simple synchronization of the captured ultrasound images. The synchronization may be, for example, with the patient's breathing or heartbeat. In this way it is possible to remove, from the ultrasound image, the effect due to the movements induced by breathing or heartbeat.

Furthermore, the electronic control of the transducers or ultrasound sensors 11 of the electronic scanning probe 1 allows having stratigraphic images of the organ to be investigated. In fact, by managing the delay in emitting and receiving the ultrasound signals of portions of the two-dimensional matrix of sensors 11, it is possible to capture images of tissue portions lying at a given depth with respect to the probe surface, i.e. at a given distance from the surface of the endocavity probe 1.

In some methods of use it is possible, for example, to reproduce the image of a section of the organ under investigation (in the specific example, the prostate) taken along an ideal cylindrical surface coaxial with the probe.

However, the possibility of controlling the delays in emitting and receiving the ultrasound signals of sensors lying on the cylindrical surface of the probe allows also capturing, alternatively, plane stratigraphic images, instead of cylindrical images, wherein the various points of the image are on a same plane and, thus, at variable distance from the ultrasound sensors 11 of adjacent longitudinal arrays.

With an endocavity probe and a dummy probe, i.e. a passive element having the same outer surface as the endocavity probe, it is possible to implement imaging methods that may have the features defined in the clauses below, for capturing images of organs of a human or animal body in vivo, which are adjacent to, i.e. associated with, a cavity of the body.

A cavity of the human or animal body "adjacent to" or "associated with" an organ means a cavity crossing, bordering on, or arranged close to, the organ whose images shall be captured, and thus a cavity adapted for the insertion of an endocavity probe adapted to capture images of the organ, specifically an endocavity ultrasound probe for capturing ultrasound images.

## Claims

1. A set comprising:
an endorectal ultrasound probe (1) comprising an elongated body (3), with a convex curved outer surface, extending along a longitudinal extension (A-A)of the elongated body (3); wherein at least an array of ultrasound sensors (11) is provided on the elongated body (3), on the curved surface to emit and receive ultrasound waves; and
a dummy endorectal probe (30), having an outer surface of shape and dimension equal to the shape and dimension of the ultrasound probe (1); wherein the material, of which the dummy probe (30) is made, is selected such as not to disturb the acquisition of data, related to chemical physical features of biological tissues of a patient under investigation by a high-definition imaging machine, such as a magnetic resonance machine.

2. The set of claim 1, wherein the dummy probe (30) is devoid of ultrasound sensors.

3. The set of claim 1 or 2, wherein the dummy probe (30) comprises at least a reference element that can be detected by means of a high-resolution imaging technique different than the ultrasound imaging.

4. The set of claim 1, 2, or 3, wherein the dummy probe (30) comprises a hollow inner volume, adapted to house a structure adapted to be inserted into, and removed from, the hollow inner volume and having at least a reference element that is detectable by means of a high-resolution imaging technique different than the ultrasound imaging.

5. The set of any one of the previous claims, wherein said high-resolution imaging technique comprises: magnetic resonance, nuclear magnetic resonance, conventional computerized axial tomography or cone beam computerized tomography, positron emission tomography.

6. The set of one or more of the previous claims, wherein the ultrasound probe (1) is an electronic scanning probe, comprising a two-dimensional matrix of ultrasound sensors (11) that can be electronically controlled.

7. A computer program comprising instructions which, when the program is executed by a computer, cause the computer to carry out a method for merging images of a prostate in vivo, the method comprising the following steps:
storing a first image of the prostate, deformed by means of a dummy endorectal probe (30) according to claim 1, inserted into the prostate, the first image being captured through a first, high-definition imaging machine, such as a magnetic resonance machine;
storing a second image of the organ captured through a endorectal ultrasound probe according to claim 1, inserted into the prostate;
superimposing and combining the first image and the second ultrasound image.

8. The computer program of claim 7, wherein the first image is a high-resolution image.

9. The computer program of claim 7 or 8, wherein the first image is captured by means of a first imaging technique selected from the group comprising: magnetic resonance, nuclear magnetic resonance, computerized axial tomography, cone beam computerized axial tomography, positron tomography.

10. The computer program of one or more of claims 7 to 9, wherein the first image is constituted by a plurality of two-dimensional images.

11. The computer program of one or more of claims 7 to 10, wherein the second image is constituted by a plurality of two-dimensional images.

## Patentansprüche

1. Set mit:
einer Endorektal-Ultraschallsonde (1) mit einem länglichen Körper (3) mit einer konvex gekrümmten Außenfläche, die sich entlang einer Längsausdehnung (A-A) des länglichen Körpers (3) erstreckt, wobei zumindest eine Anordnung von
Ultraschallsensoren (11) auf dem länglichen Körper (3) auf der gekrümmten Fläche vorgesehen ist, um Ultraschallwellen zu emittieren und zu empfangen, und
einer Dummy-Endorektal-Sonde mit einer Außenfläche einer Form und einer Abmessung, die gleich der Form und der Abmessung der Ultraschall-Sonde (1) ist, wobei das Material, aus dem die Dummy-Sonde (30) gefertigt ist, so ausgewählt ist, um Aufnahmedaten nicht zu stören, die sich auf chemische, physikalische Merkmale von biologischem Gewebe des Patienten, der untersucht wird, durch eine hochauflösende bildgebende Maschine wie einer Magnetresonanz-Maschine beziehen.

2. Set nach Anspruch 1, wobei die Dummy-Sonde (30) keine Ultraschallsensoren aufweist.

3. Set nach Anspruch 1 oder 2, wobei die Dummy-Sonde (30) mindestens ein Bezugselement aufweist, das mittels einer hochauflösenden Abbildungstechnik erfasst werden kann, die sich von Ultraschall-Bildgebung unterscheidet.

4. Set nach Anspruch 1, 2 oder 3, wobei die Dummy-Sonde (30) ein hohles inneres Volumen aufweist, das ausgebildet ist, um eine Struktur aufzunehmen, die ausgebildet ist, um in das hohle innere Volumen eingebracht und daraus entfernt zu werden, und mit mindestens einem Bezugselement, das mittels einer hochauflösenden Abbildungstechnik erfasst werden kann, die sich von der Ultraschall-Abbildungstechnik unterscheidet.

5. Set nach einem der vorstehenden Ansprüche, wobei die hochauflösende Abbildungstechnik aufweist: Magnetresonanz, Magnet-Kernresonanz, konventionelle axiale Computertomografie oder Kegelstrahl-Computertomografie, Positronen-Emissions-Tomografie.

6. Set nach einem oder mehreren der vorstehenden Ansprüche, wobei die Ultraschall-Sonde (1) eine elektronische Abtast-Sonde ist, die eine zweidimensionale Matrix von UltraschallSensoren (11) aufweist, die elektronisch gesteuert werden können.

7. Computerprogramm mit Befehlen, die, wenn das Programm durch einen Computer ausgeführt wird, den Computer veranlassen, um ein Verfahren zum Verschmelzen von Bildern einer Prostata in vivo durchzuführen, und die folgenden Schritte aufweist:
Speichern eines ersten Bildes der Prostata, die mittels einer Dummy-Endorektal-Sonde (30) nach Anspruch 1 deformiert wurde, die in die Prostata eingebracht wurde, wobei das erste Bild durch eine erste hochauflösende Bildgebungsmaschine wie einer Magnetresonanz-Maschine aufgenommen wurde,
Speichern eines zweiten Bildes des Organs, das durch eine Endorektal-Sonde nach Anspruch 1 aufgenommen wurde, die in die Prostata eingebracht wurde,
Überlagerung und Kombination des ersten und des zweiten Ultraschallbildes.

8. Computerprogramm nach Anspruch 7, wobei das erste Bild ein hochauflösendes Bild ist.

9. Computerprogramm nach Anspruch 7 oder 8, wobei das erste Bild mittels einer ersten Abbildungstechnik aufgenommen wird, die ausgewählt ist aus der Gruppe, die aufweist:
Magnetresonanz, Kern-Magnetresonanz, axiale Computertomografie, axiale Kegelstrahl-Computertomografie, Positronentomografie.

10. Computerprogramm nach einem oder mehreren der Ansprüche 7 bis 9, wobei das erste Bild aus einer Anzahl von zweidimensionalen Bildern aufgebaut ist.

11. Computerprogramm nach einem oder mehreren der Ansprüche 7 bis 10, wobei das zweite Bild aus einer Anzahl von zweidimensionalen Bildern aufgebaut ist.

## Revendications

1. Un ensemble comprenant :
une sonde échographique endo-rectale (1) comprenant un corps allongé (3), avec une surface externe incurvée et convexe, s'étendant le long d'une extension longitudinale (A-A) du corps allongé (3) ; dans laquelle il est prévu au moins un arrangement de détecteurs d'ultrasons (11) sur le corps allongé (3), sur la surface incurvée pour émettre et recevoir des ondes échographiques ; et
une sonde endo-rectale factice (30), ayant une surface externe dont la forme et la dimension sont égales à la forme et la dimension de la sonde échographique (1) ; le matériau dont est faite la sonde factice (30) étant choisi de manière à ce qu'il ne perturbe pas l'acquisition de données relatives à des caractères physico-chimiques des tissus biologiques d'un patient en cours d'examen au moyen d'une machine d'imagerie à haute définition, telle qu'une machine à résonance magnétique.

2. L'ensemble selon la revendication 1, dans lequel la sonde factice (30) est dépourvue de détecteurs d'ultrasons.

3. L'ensemble selon la revendication 1 ou 2, dans lequel la sonde factice (30) comprend au moins un élément de référence qui peut être détecté par une technique d'imagerie à haute résolution différente de l'imagerie par ultrasons.

4. L'ensemble selon la revendication 1, 2 ou 3, dans lequel la sonde factice (30) comprend un volume interne creux, apte à loger une structure apte à être introduite à l'intérieur du volume interne creux et à en être retirée, et ayant au moins un élément de référence qui est détectable au moyen d'une technique d'imagerie à haute résolution différente de l'imagerie par ultrasons.

5. L'ensemble selon l'une quelconque des revendications précédentes, dans lequel ladite technique d'imagerie à haute résolution comprend : la résonance magnétique, la résonance magnétique nucléaire, la tomographie axial conventionnelle calculée par ordinateur ou la tomographie à faisceau conique calculée par ordinateur et la tomographie par émission de positrons.

6. L'ensemble selon l'une ou plusieurs des revendications précédentes, dans lequel la sonde échographique (1) est une sonde à balayage électronique comprenant une matrice bidimensionnelle de détecteurs d'ultrasons (11) qui peuvent être commandés électroniquement.

7. Un programme d'ordinateur comprenant des instructions qui, lorsque le programme est exécuté par un ordinateur, font en sorte que l'ordinateur exécute un procédé de fusionnement d'images d'une prostate in vivo, ce procédé comprenant les étapes suivantes :
stockage d'une première image de la prostate, déformée au moyen d'une sonde endo-rectale factice (30) selon la revendication 1, introduite dans la prostate, la première image étant captée par l'intermédiaire d'une première machine d'imagerie à haute définition, telle qu'une machine à résonance magnétique ;
stockage d'une deuxième image de l'organe captée par l'intermédiaire d'une sonde à ultrasons endo-rectale selon la revendication 1, introduite dans la prostate ;
superposition et combinaison de la première image et de la deuxième image obtenue par ultrasons.

8. Le programme d'ordinateur selon la revendication 7, dans lequel la première image est image à haute résolution.

9. Le programme d'ordinateur selon la revendication 7 ou 8, dans lequel la première image est captée au moyen d'une première technique d'imagerie choisie dans le groupe comprenant : la résonance magnétique, la résonance magnétique nucléaire, la tomographie axiale par ordinateur, la tomographie axiale à faisceau conique par ordinateur, la tomographie par positrons.

10. Le programme d'ordinateur selon l'une ou plusieurs des revendications 7 à 9, dans lequel la première image est constituée d'une pluralité d'images bidimensionnelles.

11. Le programme d'ordinateur selon l'une ou plusieurs des revendications 7 à 10, dans lequel la deuxième image est constituée d'une pluralité d'images bidimensionnelles.
